# EUROPEAN PATENT APPLICATION

(11) **EP 4 434 366 A1**
(43) Date of publication of application: **25.09.2024**
(21) Application number: 21964773.2
(22) Date of filing: 19.11.2021
(51) Int. Cl.: A24F 40/465, A24F 40/51

(54) **INHALATION DEVICE**

(71) Applicant: Japan Tobacco Inc., Tokyo 105-6927 (JP)
(72) Inventor: MIZUGUCHI, Kazuma, Tokyo 130-8603 (JP); NAKANO, Takuma, Tokyo 130-8603 (JP)
(74) Representative: Hoffmann Eitle
(86) International application number: PCT/JP2021/042549
(87) International publication number: WO 2023/089758

(57) **Abstract**

Provided is an inhalation device comprising: an electromagnetic induction source 50 is disposed at a portion where a cylindrical container 110 which is filled with an aerosol source, having a bottom section 112 is inserted, and heats a side surface of the container 110 by means of induction heating; a temperature sensor 70 that is disposed at a position facing the bottom section 112 of the container 110 and detects the temperature of the container 110; and an end cap 80 that causes the bottom section 112 of the container 110 to abut the temperature sensor 70.

## Description

### Technical Field

The present invention relates to an inhalation device.

### Background Art

For example, the device described in PTL 1 includes a heating component body for heating a smoking material. The heating component body is an induction-heating component body that performs heating by induction heating, and includes a susceptor. The susceptor is a single tube-shaped member made of a material that can be induction heated. In other words, the susceptor generates heat when being present close to a varying magnetic field. In addition, the heating component body includes a first temperature detection element and a second temperature detection element. The first temperature detection element and the second temperature detection element are disposed to be in contact with the susceptor and detect the temperature of the susceptor. The smoking material includes, for example, a material that provides, when being heated, a volatile component in an aerosol form typically. The smoking material has, for example, a form of a rod that is insertable into the device.

### Citation List

### Patent Literature

PTL 1: Japanese Unexamined Patent Application Publication No. 2021-122

### Summary of Invention

### Technical Problem

The device described in PTL 1 is heated in a state in which the rod-shaped smoking material is inserted into the device. Then, when the smoking material is, for example, a smoking material that is formed by wrapping shredded tobacco as an aerosol source with paper so as to be molded into a rod shape such that the shredded tobacco is exposed at a tip portion of the rod shape, a portion (hereinafter also referred to as "waste of tobacco leaves and the like") of heated shredded tobacco separated from a body remains in the device after inhalation. Waste of tobacco leaves and the like is required to be removed for maintenance of the device. Thus, if the amount of the waste of the tobacco leaves and the like is large, the maintenance of the device requires much time and labor.

Meanwhile, since the aerosol source is heated by heat that is generated in a member (the susceptor in PTL 1) that heats the aerosol source, the temperature of the member that heats the aerosol source may be detected and heating control of this member may be performed on the basis of the detected temperature to cause the temperature of the aerosol source to be an appropriate temperature. For the above, it is desirable to highly accurately detect the temperature of the member that heats the aerosol source.

The present invention provides an inhalation device that can suppress time and labor for maintenance of the device and can highly accurately detect the temperature of a member that heats an aerosol source.

### Solution to Problem

A first feature of the present invention completed for such a purpose is an inhalation device including: an electromagnetic induction source that is disposed at a portion into which a container is to be inserted, and that heats a side surface of the container by induction heating, the container having a tubular shape and a bottom and being filled with an aerosol source; a temperature sensor that is disposed at a position facing the bottom of the container and that detects a temperature of the container; and an abutting member that causes the bottom of the container to abut onto the temperature sensor.

A second feature is an inhalation device including: a non-magnetic member that has a tubular shape and that is disposed around a container, the container having a tubular shape and a bottom and being filled with an aerosol source; an electromagnetic induction source that heats the container by induction heating, the electromagnetic induction source being constituted by a conductive wire that is wound into a helical shape around an outer peripheral surface of the non-magnetic member; and a temperature sensor that includes a detector that detects a temperature of the container, and a lead wire that outputs a detection value, the detector being disposed in an inside of the non-magnetic member to overlap a region in which the container is provided in a centerline direction of the non-magnetic member, the lead wire passing between portions of the conductive wire of the electromagnetic induction source to extend from the detector to an outer side of the electromagnetic induction source.

A third feature is an inhalation device including: a non-magnetic member that has a tubular shape and that is disposed around a container, the container having a tubular shape and a bottom and being filled with an aerosol source; an electromagnetic induction source that is disposed around the non-magnetic member and that heats the container by induction heating; and a temperature sensor that includes a detector that detects a temperature of the container, and a lead wire that outputs a detection value, the detector being disposed in an inside of the non-magnetic member to overlap a region in which the container is provided in a centerline direction of the non-magnetic member, the lead wire passing an outer side in the centerline direction with respect to the electromagnetic induction source to extend from the detector to the outer side of the electromagnetic induction source.

A fourth feature may further include a substrate to which an end portion of the lead wire is connected, in which, at a position closer than the electromagnetic induction source to the substrate in the centerline direction, the lead wire may extend to the outer side of the electromagnetic induction source.

A fifth feature is an inhalation device including: an electromagnetic induction source that heats a container by induction heating, the container having a tubular shape and a bottom and being filled with an aerosol source, the electromagnetic induction source being disposed at an insertion portion into which the container is to be inserted; an opening-closing member that changes between a state of covering an opening of the insertion portion and a state of opening the opening, the opening-closing member having, in the state of covering, an outlet for aerosol that is generated as a result of heating of the aerosol source; and a temperature sensor that is attached to the opening-closing member and that detects a temperature of the container.

As a sixth feature, the container may include a tubular portion that has a tubular shape, and a protrusion at a portion on a side opposite to the bottom in a centerline direction, the protrusion protruding outward from the tubular portion, and the temperature sensor may include a detector that detects a temperature of the container, and a lead wire that outputs a detection value, the detector being disposed to face and to come into contact with the protrusion in the centerline direction, the lead wire passing an outer side of the electromagnetic induction source and being connected to a substrate.

### Advantageous Effects of Invention

According to the first feature, it is possible to suppress time and labor for maintenance of the inhalation device and possible to highly accurately detect the temperature of the container filled with the aerosol source.

According the second feature, it is possible to suppress time and labor for maintenance of the inhalation device and possible to highly accurately detect the temperature of the container filled with the aerosol source.

According to the third feature, it is possible to suppress time and labor for maintenance of the inhalation device and possible to highly accurately detect the temperature of the container filled with the aerosol source.

According to the fourth feature, it is possible to shorten the lead wire.

According to the fifth feature, it is possible to suppress time and labor for maintenance of the inhalation device and possible to detect the temperature of the container filled with the aerosol source.

According to the sixth feature, it is possible to further highly accurately detect the temperature of the container.

### Brief Description of Drawings

[Fig. 1] Fig. 1 is a perspective view illustrating one example of a state in which a component that constitutes an inhalation device according to a first embodiment is dismantled.
[Fig. 2] Fig. 2 is one example of a sectional view illustrating a general configuration of the inhalation device according to the first embodiment.
[Fig. 3] Fig. 3 is a diagram schematically illustrating one example of a general configuration of the inhalation device according to the first embodiment.
[Fig. 4] Fig. 4 is one example of a sectional view illustrating one example of a general configuration of an inhalation device according to a second embodiment.
[Fig. 5] Fig. 5 is a view illustrating one example of general configurations of a support member and a container according to a modification.
[Fig. 6] Fig. 6 is one example of a sectional view illustrating one example of a general configuration of an inhalation device according to a third embodiment.
[Fig. 7] Fig. 7 is one example of a sectional view illustrating one example of a general configuration of an inhalation device according to a fourth embodiment.
[Fig. 8] Fig. 8 is one example of a sectional view illustrating one example of a general configuration of an inhalation device according to a fifth embodiment.
[Fig. 9] Fig. 9 is one example of a sectional view illustrating one example of a general configuration of an inhalation device according to a sixth embodiment.

### Description of Embodiments

Hereinafter, embodiments according to the present invention will be described in detail with reference to the accompanying drawings.

### <First Embodiment>

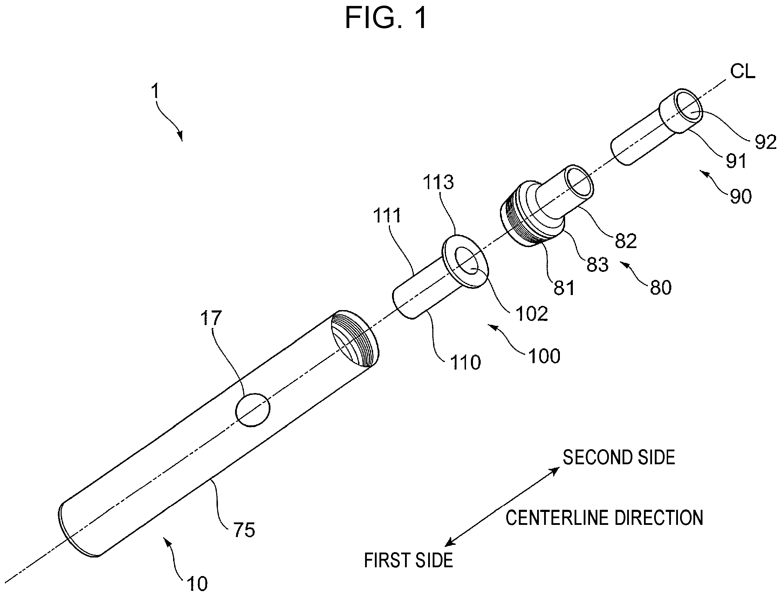

Fig. 1 is a perspective view illustrating one example of a state in which a component that constitutes an inhalation device 1 according to a first embodiment is dismantled.

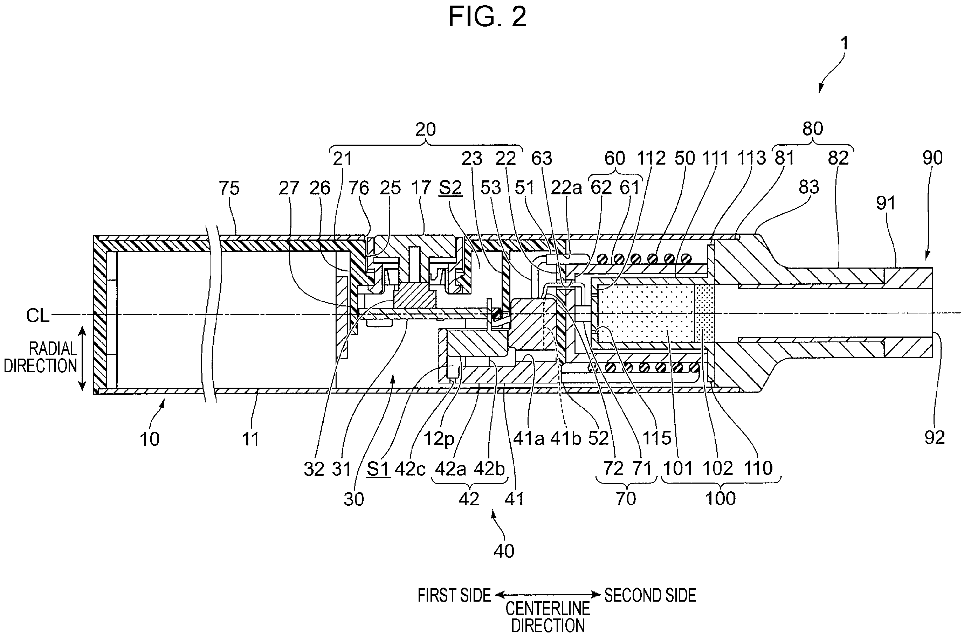

Fig. 2 is one example of a sectional view illustrating a general configuration of the inhalation device 1 according to the first embodiment.

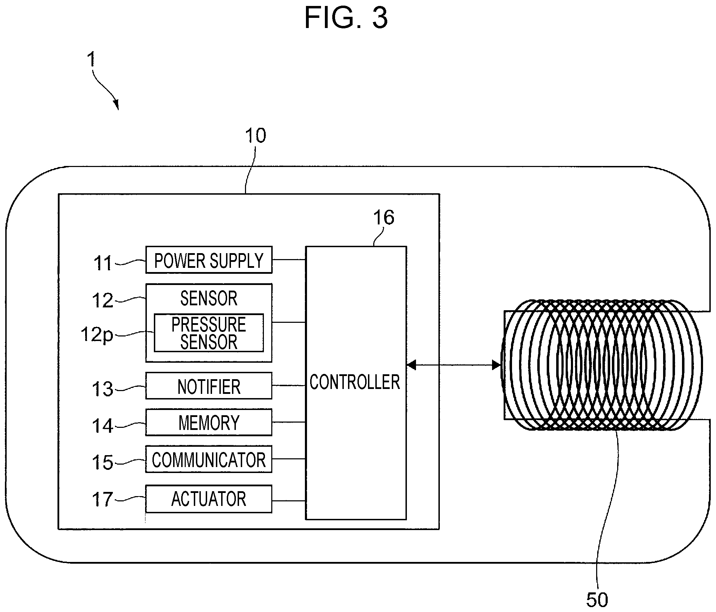

Fig. 3 is a diagram schematically illustrating one example of a general configuration of the inhalation device 1 according to the first embodiment.

The inhalation device 1 according to the first embodiment is a device that generates a material that is to be inhaled by a user. Following description is based on that the material generated by the inhalation device 1 is aerosol. Alternatively, the material generated by the inhalation device 1 may be a gas.

The inhalation device 1 generates aerosol by heating a substrate 100 containing an aerosol source by induction heating (IH).

The inhalation device 1 includes a power supply unit 10, an electromagnetic induction source 50, a support member 60 that supports the substrate 100, and a temperature sensor 70 that detects the temperature of the substrate 100. In addition, the inhalation device 1 includes a cover 75 that covers outer circumferences of the power supply unit 10, the electromagnetic induction source 50, the support member 60, and the like; a mouthpiece 90; and an end cap 80 that accommodates a portion of the mouthpiece 90. In a state in which the substrate 100 is supported by the support member 60 in the inhalation device 1, inhalation is performed by a user.

The cover 75 has a cylindrical shape. The power supply unit 10, the electromagnetic induction source 50, and the mouthpiece 90 are arranged side by side in the direction of a centerline CL of the cylindrical shape. In the following description, the direction of the centerline CL may be referred to as "centerline direction". In addition, in the centerline direction, the side where the power supply unit 10 is disposed may be referred to as "first side", and the mouthpiece 90 side may be referred to as "second side". Further, in view in the centerline direction, the direction intersecting the centerline CL may be referred to as "radial direction", and the direction around the centerline CL may be referred to as "circumferential direction".

### (Power Supply Unit 10)

As illustrated in Fig. 3, the power supply unit 10 includes a power supply 11, a sensor 12, a notifier 13, a memory 14, a communicator 15, a controller 16, and an actuator 17 that is operable by a user.

The power supply 11 stores electric power. Then, the power supply 11 supplies the electric power to each of structural elements of the inhalation device 1. The power supply 11 can be constituted by, for example, a rechargeable battery, such as a lithium ion secondary battery. The power supply 11 may be charged by being connected to an external power supply by a USB (Universal Serial Bus) cable or the like. In addition, the power supply 11 may be charged in a state of being not connected to a power-transmission-side device, by wireless power transmission technology. Additionally, only the power supply 11 may be detachable from the inhalation device 1 and may be replaced with a new power supply 11.

The sensor 12 detects various items of information on the inhalation device 1. As one example, the sensor 12 includes a pressure sensor 12p, such as a microphone condenser. Then, the sensor 12 outputs detected information to the controller 16. For example, when the pressure sensor 12p detects a numerical value resulted from inhalation by a user, the sensor 12 outputs information indicating that inhalation has been performed by the user to the controller 16.

The notifier 13 notifies the user of information. As one example, the notifier 13 is constituted by a light-emitting device, such as an LED (Light Emitting Diode). In this case, the notifier 13 emits light in different patterns of light in each of a case where the state of the power supply 11 requires charging, a case where the power supply 11 is under charging, a case where an abnormality has occurred in the inhalation device 1, and the like. The patterns of light here are concepts including colors, timing of turning on/off, and the like. The notifier 13 may be constituted by, in addition or as an alternative to the light-emitting device, a display device that displays an image, a sound output device that outputs a sound, a vibration device that vibrates, and the like.

The memory 14 stores various items of information for an operation of the inhalation device 1. The memory 14 is constituted by, for example, a nonvolatile storage medium, such as a flash memory. One example of the information stored in the memory 14 is information on an OS (Operating System) of the inhalation device 1, such as contents of control of each of structural elements by the controller 16. Another example of the information stored in the memory 14 is information on inhalation by a user, such as the number of times of inhalation, inhalation time, and an accumulated inhalation time period.

The communicator 15 is a communication interface for transmitting and sending information between the inhalation device 1 and another device. The communicator 15 performs communication in conformity with a wired or wireless optional communication standard. As such a communication standard, for example, a wireless LAN (Local Area Network), a wired LAN, Wi-Fi (registered trademark), Bluetooth (registered trademark), or the like is employable. As one example, the communicator 15 transmits information on inhalation performed by a user to a smartphone to cause the smartphone to display the information on the inhalation performed by the user. As another example, the communicator 15 receives information on a new OS from a server to update OS information stored in the memory 14.

The controller 16 functions as an arithmetic processing unit and a control unit and controls the whole operation in the inhalation device 1 in accordance with various programs. The controller 16 is implemented by, for example, a CPU (Central Processing Unit) , an electronic circuit, such as a microprocessor. In addition, the controller 16 may include a ROM (Read Only Memory) that stores programs, arithmetic operation parameters, and the like that are to be used, and a RAM (Random Access Memory) that temporarily stores parameters and the like that change, as appropriate. The inhalation device 1 executes various processes on the basis of control by the controller 16. Examples of the processes controlled by the controller 16 are supplying of electric power from the power supply 11 to each of the other structural elements, charging of the power supply 11, detection of information by the sensor 12, notification of information by the notifier 13, storing and retrieving of information by the memory 14, and transmitting and receiving of information by the communicator 15. The controller 16 also controls input of information to each of the structural elements and other processes that are to be executed by the inhalation device 1, such as processes based on information output from the structural elements.

The actuator 17 is constituted by a button-type switch or the like. However, the actuator 17 may be constituted by a touch panel or the like. The actuator 17 is provided in a state of being exposed from a surface of the cover 75. The actuator 17 outputs information operated by a user to the controller 16. For example, when a predetermined starting operation is performed with respect to the actuator 17 in a state in which the power supply unit 10 is in a power OFF state, the actuator 17 outputs a command to start the power supply unit 10 to the controller 16. When obtained this starting command, the controller 16 starts the power supply unit 10. As an example, the predetermined starting operation using the actuator 17 is three times of quick pressing of the actuator 17.

As illustrated in Fig. 2, the power supply unit 10 includes a holder 20 that holds the power supply 11 and the like; a substrate module 30 that constitutes the controller 16 and the like; and a sensor cover 40 that covers the periphery of the pressure sensor 12p.

### [Holder 20]

The holder 20 includes a base portion 21 that has a semi-cylindrical shape having the centerline CL as the centerline thereof; a disk-shaped portion 22 that has a disk shape and that is provided at an end portion of the base portion 21 on the second side in the centerline direction; and a holding portion 23 that sandwiches an attachment portion 41, described later, of the sensor cover 40 in cooperation with the disk-shaped portion 22. The holder 20 is, for example, made of a resin material integrally.

The base portion 21 has a semi-cylindrical shape having the centerline CL as the centerline thereof and is capable of receiving the power supply 11. Note that the base portion 21 may have a shape other than a semi-cylindrical shape as long as an opening for receiving the power supply 11 opens in the base portion 21.

An opening 25 that opens to expose the actuator 17 from the cover 75 is formed at a portion of the base portion 21 on the second side in the centerline direction. The opening 25 extends in the radial direction through a portion of the base portion 21 in the circumferential direction.

The base portion 21 includes a protrusion 26 protruding from an outer peripheral portion toward the centerline CL to surround the periphery of the opening 25.

The base portion 21 includes, on the first side in the centerline direction with respect to the protrusion 26, a dividing wall 27 that divides the base portion 21 into a portion that holds the power supply 11 and the other portion.

The disk-shaped portion 22 has a plurality of through holes 22a extending in the centerline direction. One end portion 51, described later, of the electromagnetic induction source 50 passes through a through hole 22a toward the substrate module 30. In addition, a lead wire 72, described later, of the temperature sensor 70 passes through the through hole 22a toward the substrate module 30.

The holding portion 23 has a semi-disk shape protruding from the inner peripheral surface of the base portion 21 toward the centerline CL.

### [Substrate Module 30]

The substrate module 30 includes a substrate 31 and a switch element 32 mounted on the substrate 31.

The substrate 31 is disposed to be parallel to the centerline direction and to be orthogonal to the protrusion 26. The substrate 31 is, for example, fixed to the base portion 21 of the holder 20 by a screw. The substrate 31 is connected to the power supply 11 via a connection wire (not illustrated). In addition, a MCU (Micro Controller Unit) (not illustrated) that implements the controller 16 is mounted on the substrate 31. Further, the pressure sensor 12p is mounted on the substrate 31.

The switch element 32 is disposed on a surface of the substrate 31 on the protrusion 26 side to be at a position overlapping the opening 25 in the radial direction. In the example illustrated in Fig. 2, the switch element 32 is surface-mounted on the substrate 31. However, the switch element 32 may be mounted on the substrate 31 in a state in which a connection terminal extended from the switch element 32 is inserted into a through hole of the substrate 31.

The switch element 32 is disposed at a position displaced toward the first side in the centerline direction with respect to the pressure sensor 12p. In other words, the switch element 32 is disposed at a position not overlapping the pressure sensor 12p when viewed in the radial direction from the outer side of the base portion 21.

### [Sensor Cover 40]

The sensor cover 40 is molded with a resin material, such as a silicone resin, that is softer than the holder 20 and that has elasticity. The sensor cover 40 includes the attachment portion 41 that is attached to the holder 20, and a cover portion 42 that covers the pressure sensor 12p.

The attachment portion 41 has a semi-circular pillar shape to have a centerline in the centerline direction and to be positioned on a side opposite to the base portion 21 in the circumferential direction. Then, the attachment portion 41 is fitted into a space between the disk-shaped portion 22 and the holding portion 23 of the holder 20. The attachment portion 41 has a through hole 41a extending in the centerline direction. In addition, the attachment portion 41 has, in a surface on the second side in the centerline direction, a groove 41b that causes the through hole 41a and the space between the disk-shaped portion 22 and the holding portion 23 of the holder 20 to be in communication with each other in a state in which the sensor cover 40 is attached to the holder 20.

The cover portion 42 is provided to extend from an outer peripheral portion of the attachment portion 41 toward the first side in the centerline direction and has a recessed shape to open on the centerline CL side in the radial direction. The cover portion 42 includes a plurality of projections 42b protruding from a bottom 42a having a recessed shape toward the centerline CL side. The bottom 42a has a through hole 42c extending in the radial direction and causing the inside and the outside of the recessed shape to be in communication with each other. The pressure sensor 12p is fitted to the inside of the recessed shape of the cover portion 42 until abutting onto the projections 42b. Consequently, a gap is formed between the bottom surface of the bottom 42a and the pressure sensor 12p.

As illustrated in Fig. 2, a space surrounded by the sensor cover 40 constitutes a pressure fluctuating chamber S1 in which pressure fluctuates in response to inhalation with the inhalation device 1 through the through hole 41a of the attachment portion 41 described above. In contrast, in the cover 75, a space other than the pressure fluctuating chamber S1 constitutes an atmospheric pressure chamber S2 in which atmospheric pressure acts. In the present embodiment, the power supply 11 and the substrate module 30 other than the pressure sensor 12p are accommodated in the atmospheric pressure chamber S2

### (Substrate 100)

The substrate 100 includes a substrate 101, a filter 102, and a container 110 that accommodates the substrate 101 and the filter 102.

The substrate 101 contains an aerosol source. The aerosol source is atomized by being heated and generates aerosol. The aerosol source may be, for example, a material derived from tobacco, such as a product made of shredded tobacco or a tobacco raw material that is molded into a granular form, a sheet form, or a power form. The aerosol source may contain a material that is not derived from tobacco and that is made of a plant (for example, mint, herb, and the like) other than tobacco. As one example, the aerosol source may contain a flavor component such as menthol. When the inhalation device 1 is a medical inhaler, the aerosol source may contain a medicine that is to be inhaled by a patient.

The container 110 is molded with a magnetic material. As examples of the magnetic material, iron and ferritic stainless steel can be presented. In addition, as an example, the container 110 is molded by pressing.

The container 110 includes a cylindrical portion 111 that has a cylindrical shape; a bottom 112 that closes an end portion of the cylindrical portion 111 on the first side in the centerline direction; and a protrusion 113 that protrudes outward in the radial direction from an end portion of the cylindrical portion 111 on the second side in the centerline direction to extend around the whole circumference.

The outside diameter of the cylindrical portion 111 is smaller than the inside diameter of a cylindrical portion 61, described later, of the support member 60, and the cylindrical portion 111 and the substrate 101 are accommodated in the inside of the support member 60.

The bottom 112 has a plurality of through holes 115 extending in the centerline direction. The size of each of the through holes 115 is smaller than the size of the aerosol source (for example, the size of each of the through holes 115 is 1/10 or less of the size of the aerosol source) so that the aerosol source does not easily exit to the outside through the through holes 115.

The outside diameter of the protrusion 113 is larger than the outside diameter of the cylindrical portion 61, described later, of the support member 60 and is smaller than the inside diameter of the cover 75. Then, the container 110 is accommodated in the cover 75 in a state in which the protrusion 113 is placed on an end surface of the cylindrical portion 61 of the support member 60 on the second side in the centerline direction.

### (Electromagnetic Induction Source 50)

By electromagnetic induction, the electromagnetic induction source 50 causes the container 110 of the substrate 100 to produce heat. The electromagnetic induction source 50 is constituted by a coil-shaped conductive wire and is disposed to be wound around the outer circumference of the support member 60. As an example, the conductive wire is a polyurethane copper wire including a conductor and an electrically insulative coating material (resin) that is applied to the conductor and baked. The one end portion 51 of the electromagnetic induction source 50 passes through the through hole 22a formed in the disk-shaped portion 22 of the holder 20 and is connected to the substrate 31 via a lead wire 53. In addition, another end portion 52 of the electromagnetic induction source 50 passes through a gap formed between the disk-shaped portion 22 of the holder 20 and the cover 75, extends toward the first side in the centerline direction, and is connected to the substrate 31 via, for example, a lead wire (not illustrated). Note that the one end portion 51 and the other end portion 52 may be each directly connected to the substrate 31.

The electromagnetic induction source 50 generates a magnetic field when being supplied with alternating current from the power supply 11. The electromagnetic induction source 50 is disposed at a position at which the container 110 of the substrate 100 supported by the support member 60 overlaps the generated magnetic field. Therefore, when a magnetic field is generated in a state in which the substrate 100 is supported by the support member 60, eddy current is generated in the container 110, and Joule heat is generated. Then, the aerosol source contained in the substrate 100 is heated to be atomized by the Joule heat and generates aerosol. As one example, to generate aerosol, electric power may be supplied when a predetermined operation is performed with respect to the actuator 17 by a user. When the temperature of the substrate 100 induction heated by the electromagnetic induction source 50 reaches a predetermined temperature, inhalation is enabled for a user. Thereafter, electric power supply may be stopped when the number of times of detection that a pressure detected by the pressure sensor 12p is, for example, less than a predetermined value reaches a predetermined number of times.

### (Support Member 60)

The support member 60 is molded with a non-magnetic material. As examples of the non-magnetic material, a synthetic resin and austenitic stainless steel can be presented. The support member 60 includes the cylindrical portion 61 that has a cylindrical shape and a bottom 62 that closes an end portion of the cylindrical portion 61 on the first side in the centerline direction. The bottom 62 has a through hole 63 extending in the centerline direction.

The electromagnetic induction source 50 wound into a helical shape is disposed on the outer peripheral surface of the cylindrical portion 61.

Note that, although the cylindrical portion 61 and the bottom 62 are integrated in the support member 60 illustrated in Fig. 2, the cylindrical portion 61 and the bottom 62 may be separate bodies. Further, the support member 60 may include only a disk-shaped member corresponding to the bottom 62 and does not need to include a cylindrical member, which corresponds to the cylindrical portion 61, that is disposed on the inner side of the electromagnetic induction source 50.

### (Temperature Sensor 70)

As an example, the temperature sensor 70 is a thermistor that includes a detector 71 and the lead wire 72. In the example illustrated in Fig. 2, the detector 71 of the temperature sensor 70 is disposed on the second side of the bottom 62 of the support member 60 in the centerline direction. As an example, the detector 71 is bonded to the bottom 62 with a thermally conductive adhesive, for example, epoxy or the like. The lead wire 72 passes through the through hole 63 of the bottom 62 of the support member 60 and the through holes 22a of the disk-shaped portion 22, and two end portions provided at a tip thereof are each connected to the substrate 31.

A detection signal of the temperature sensor 70 is input to the controller 16. Then, on the basis of a temperature detected by the temperature sensor 70, the controller 16 controls a driver (not illustrated) mounted on the substrate 31 to vary high-frequency current that is to be supplied to the electromagnetic induction source 50. As described above, the controller 16 controls the temperature of the substrate 100 to be a target temperature that is prescribed in a heating profile previously stored in a ROM. Note that the temperature sensor 70 may be a thermocouple.

### (Cover 75)

The cover 75 has a cylindrical shape having the centerline CL as the centerline thereof. The holder 20 is inserted into the cover 75. A portion of the cover 75, the portion facing the opening 25 of the base portion 21 of the holder 20, has an exposure hole 76 for exposing the actuator 17.

The actuator 17 is accommodated in the exposure hole 76 of the cover 75 and the opening 25 of the holder 20. The actuator 17 is configured to be movable in the radial direction in a state of being supported by the protrusion 26. By moving toward the centerline CL in the radial direction, the actuator 17 presses the switch element 32. Note that the actuator 17 is not limited to move in the radial direction and may move, for example, slidingly in the centerline direction. The actuator 17 may be a touch sensor.

### (End Cap 80)

The end cap 80 includes a first cylindrical portion 81 that has a cylindrical shape and that is fitted to the inner side of an opening of the cover 75 on the second side, and a second cylindrical portion 82 that has a cylindrical shape and that is provided on the outer side of the cover 75.

A portion of the first cylindrical portion 81 on the cover 75 side is fitted to the inner side of an opening of the cover 75 on the second side, and the first cylindrical portion 81 includes a flange 83 that abuts onto an end surface of the cover 75. The diameter of the outer peripheral surface of the second cylindrical portion 82 is smaller than the diameter of the outer peripheral surface of the first cylindrical portion 81, and the diameter of the inner peripheral surface of the second cylindrical portion 82 is identical to the diameter of the inner peripheral surface of the first cylindrical portion 81.

As an example, the end cap 80 is molded with a resin or a metal.

In the example illustrated in Fig. 1, the end cap 80 is fixed to the cover 75 by screw fastening of a male screw formed on the outer peripheral surface of the first cylindrical portion 81 and a female screw formed on the inner peripheral surface of the cover 75. Note that the end cap 80 may be fixed to the cover 75 by the first cylindrical portion 81 being press-fitted to the cover 75.

As a result of the end cap 80 being attached to the cover 75, an end portion of the first cylindrical portion 81 on the first side in the centerline direction comes into contact with the container 110 of the substrate 100, and the container 110 is pressurized toward the first side. Consequently, a contact state of the bottom 112 of the container 110 and the detector 71 of the temperature sensor 70 is kept. In addition, a movement of the substrate 100 toward the second side in the centerline direction is suppressed. Note that, although the protrusion 113 of the container 110 is in contact with an end portion of the cylindrical portion 61 of the support member 60 on the second side in the centerline direction in the example illustrated in Fig. 2, the protrusion 113 and the cylindrical portion 61 may be not in contact with each other.

### (Mouthpiece 90)

The mouthpiece 90 is a cylindrical member, and a portion of the mouthpiece 90 on the first side in the centerline direction is fitted to the inner side of the end cap 80. The mouthpiece 90 includes a flange 91 that abuts onto an end surface of the end cap 80. As an example, the mouthpiece 90 is molded with a resin.

The mouthpiece 90 is a member that is held in the mouth of a user during inhalation. The mouthpiece 90 has an air outlet hole 92. By holding the mouthpiece 90 in the mouth and inhaling, a user can take a mixture fluid of aerosol and air into the oral cavity.

Note that the mouthpiece 90 and the end cap 80 may be molded integrally with a resin.

### (Operation of Inhalation Device 1)

By inserting the container 110 filled with an aerosol source to the inner side of the electromagnetic induction source 50, in other words, into the support member 60 and induction heating the container 110 by using the electromagnetic induction source 50, a user can cause the inhalation device 1 configured as described above to be in a state in which aerosol can be inhaled. Then, when inhalation is performed by a user, the aerosol generated as a result of heating of the aerosol source accommodated in the container 110 flows in, for example, through a gap between the actuator 17 and the cover 75 and mixes with air that has passed through the through holes 22a of the disk-shaped portion 22, the through hole 63 of the bottom 62 of the support member 60, and the through holes 115 of the bottom 112 of the container 110. Then, the mixture fluid of the aerosol and the air passes through the filter 102 and is conveyed to the air outlet hole 92 formed in the mouthpiece 90.

After finishing inhalation of the aerosol, by taking out the container 110 from the inner side of the electromagnetic induction source 50 and inserting a new container 110 to the inner side of the electromagnetic induction source 50 again, a user can obtain a state in which aerosol can be inhaled again. Alternatively, a user can obtain the state in which aerosol can be inhaled again by, after taking out the heated substrate 101 and the filter 102 from the inside of the container 110 that has been taken out from the inner side of the electromagnetic induction source 50 and discarding the substrate 101 and the filter 102, inputting a new substrate 101 and a new filter 102 in the container 110 and inserting this container 110 to the inner side of the electromagnetic induction source 50 again. Then, since the container 110 is provided with the bottom 112, the heated aerosol source tends to remain in the container 110 after inhalation and suppresses remaining of a portion of the heated aerosol source on the inner side of the electromagnetic induction source 50 after the container 110 is taken out. Therefore, according to the inhalation device 1, the need to remove a portion of the heated aerosol source to perform maintenance of the inhalation device 1 is reduced, which reduces time and labor required for the maintenance, compared with, for example, a configuration in which a stick-type substrate containing an aerosol source is heated.

In addition, the inhalation device 1 includes the temperature sensor 70 that is disposed at a position facing the bottom 112 of the container 110 and that detects the temperature of the container 110, and the end cap 80 as one example of an abutting member that causes the bottom 112 of the container 110 to abut onto the temperature sensor 70. Due to the temperature sensor 70 being disposed at the position facing the bottom 112 of the container 110 and due to the end cap 80 causing the bottom 112 of the container 110 to abut onto the detector 71 of the temperature sensor 70, it is possible in the inhalation device 1 configured as described above to cause the bottom 112 of the container 110 and the detector 71 of the temperature sensor 70 to come into contact with each other and possible to detect the temperature of the container 110 highly accurately.

Therefore, according to the inhalation device 1, it is possible to suppress time and labor for maintenance of the inhalation device 1 and possible to highly accurately detect the temperature of the container 110 that heats the substrate 101 containing the aerosol source. In addition, since the temperature of the container 110 can be detected highly accurately, the controller 16 can suppress an excessive increase in the temperature difference between the temperature of the substrate 100 and a target temperature. As a result, according to the inhalation device 1, it is possible to cause the temperature of the substrate 101 packed in the container 110 to be an appropriate temperature.

### <Second Embodiment>

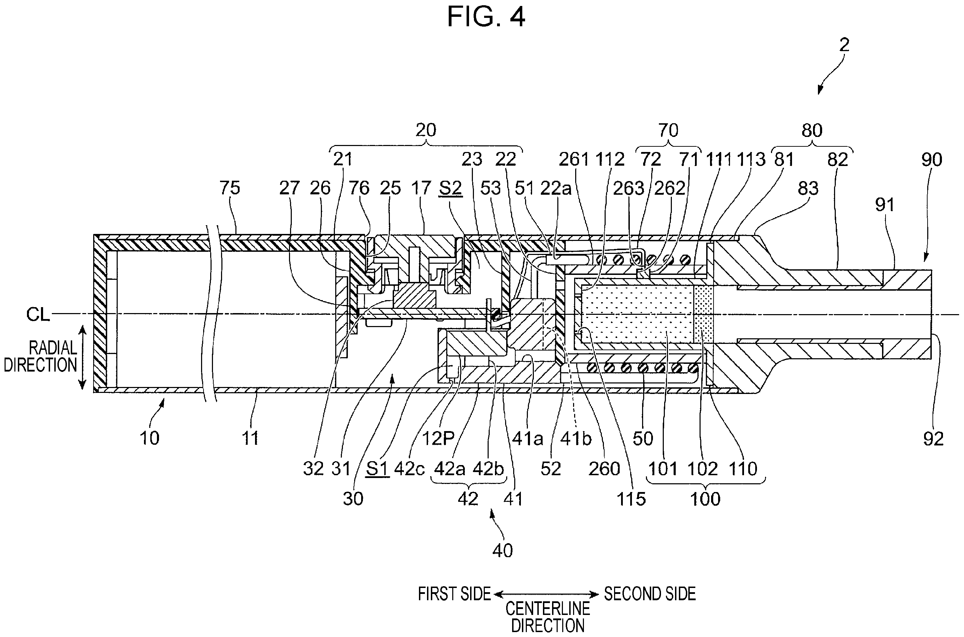

Fig. 4 is one example of a sectional view illustrating one example of a general configuration of an inhalation device 2 according to a second embodiment.

The inhalation device 2 according to the second embodiment differs from the inhalation device 1 according to the first embodiment in terms of the shape of a support member 260, which corresponds to the support member 60, and the position at which the temperature sensor 70 is provided. Hereinafter, differences from the first embodiment will be described. Identical signs are used for identical members in the first embodiment and the second embodiment, and detailed description thereof is omitted.

The support member 260 includes a cylindrical portion 261 having a cylindrical shape. A central portion of the cylindrical portion 261 in the centerline direction has a recess 262 that is recessed from the inner peripheral surface and a through hole 263 extending in the radial direction and causing the recess 262 to be in communication with the outside of the cylindrical portion 261. Then, the detector 71 of the temperature sensor 70 is disposed in the recess 262. The lead wire 72 of the temperature sensor 70 passes through the through hole 263, passes between portions of the conductive wire of the electromagnetic induction source 50 wound into a helical shape around the outer peripheral surface of the cylindrical portion 261, and passes through a gap between the outer side of the electromagnetic induction source 50 in the radial direction and the inner peripheral surface of the cover 75 and the through holes 22a of the disk-shaped portion 22. Two end portions provided at a tip of the lead wire 72 are each connected to the substrate 31.

As described above, the inhalation device 2 includes the support member 260 as one example of a non-magnetic member that has a tubular shape and that is disposed around the container 110, the container 110 having a tubular shape and the bottom 112 and being filled with the aerosol source, and the electromagnetic induction source 50 that is constituted by the conductive wire wound into the helical shape around the outer peripheral surface of the support member 260 and that heats the container 110 by induction heating. In addition, the inhalation device 2 includes the temperature sensor 70 that includes the detector 71 that detects the temperature of the container 110, and the lead wire 72 that outputs a detection value, the detector 71 being disposed in the inside of the support member 260 to overlap a region in which the container 110 is provided in the centerline direction of the support member 260, the lead wire 72 passing between portions of the conductive wire of the electromagnetic induction source 50 to extend from the detector 71 to the outer side of the electromagnetic induction source 50 in the radial direction.

In the inhalation device 2 that is configured as described, since the detector 71 is disposed in the centerline direction to overlap the region in which the container 110 is provided, the temperature sensor 70 detects the temperature of the cylindrical portion 111 of the container 110.

Therefore, according to the inhalation device 2, it is possible to suppress time and labor for maintenance of the inhalation device 2 and possible to highly accurately detect the temperature of the container 110 that heats the substrate 101 containing the aerosol source. In addition, since the temperature of the container 110 can be detected highly accurately, the controller 16 can suppress an excessive increase in the temperature difference between the temperature of the substrate 100 and a target temperature.

Further, the temperature sensor 70 detects the temperature of a central portion of the cylindrical portion 111 of the container 110, in which Joule heat is easily generated, in the centerline direction. Therefore, according to the inhalation device 2, it is possible to detect the temperature of a portion close to a portion that tends to have a highest temperature in the substrate 101 containing the aerosol source, and thus is possible to control the electric current that is to be supplied to the electromagnetic induction source 50 so that the temperature of the aerosol does not excessively increase.

Note that it is desirable to dispose the detector 71 such that the detector 71 of the temperature sensor 70 comes into contact with the outer peripheral surface of the cylindrical portion 111 of the container 110. For example, it is desirable to set a distance from the centerline CL to the detector 71 in the radial direction to be smaller than the radius of the outer peripheral surface of the cylindrical portion 111. Further, it is desirable to set the outside diameter of the protrusion 113 of the container 110 to be substantially the same as the diameter of the inner peripheral surface of the cover 75 so that the centerline of the container 110 easily coincides with the centerline CL. Alternatively, a projection protruding from the inner peripheral surface may be provided at a portion of the cylindrical portion 261 of the support member 260 on a side opposite to the recess 262 with respect to the centerline CL, and the distance between this projection and the detector 71 may be set to be smaller than the diameter of the outer peripheral surface of the cylindrical portion 111 of the container 110. Further, a portion at which a line that connects this projection and the recess 262 to each other intersects the inner peripheral surface of the cylindrical portion 261 of the support member 260 and a portion at which a line orthogonal to the centerline CL intersects the inner peripheral surface of the cylindrical portion 261 of the support member 260 may be each provided with a projection protruding from the inner peripheral surface, and the diameter of an imaginary circle drawn by these three projections and the detector 71 may be set to be smaller than the diameter of the outer peripheral surface of the cylindrical portion 111 of the container 110.

As a result of the detector 71 of the temperature sensor 70 coming into contact with the outer peripheral surface of the cylindrical portion 111 of the container 110, the temperature sensor 70 can detect the temperature of the container 110 highly accurately. In addition, due to being able to detect the temperature of the container 110 highly accurately, the controller 16 can suppress an excessive increase in the temperature difference between a target temperature and the temperature of the container 110, eventually, the temperature of the substrate 101 packed in the container 110.

### (Modification of Support Member 260 and Container 110)

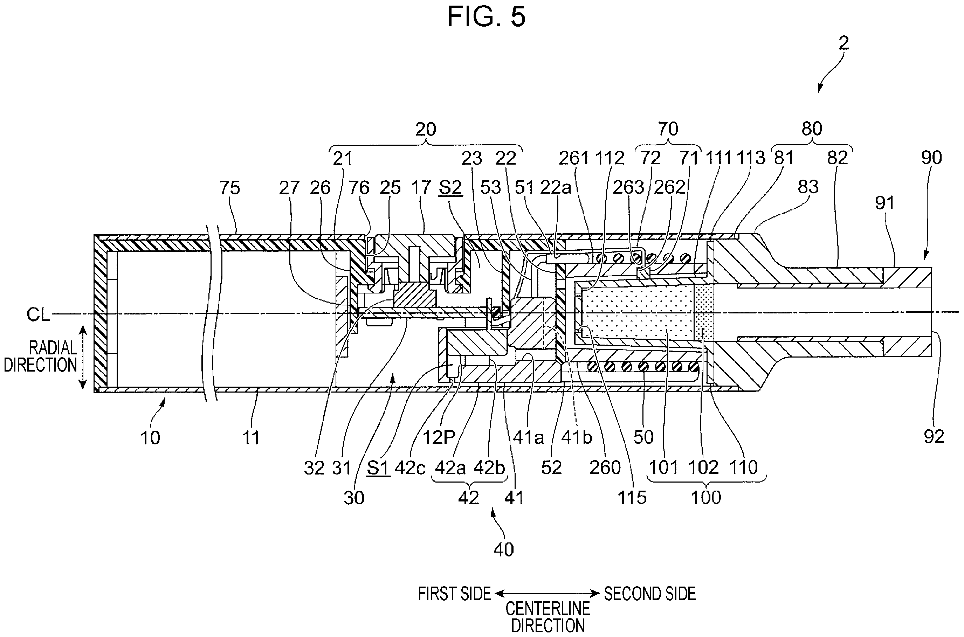

Fig. 5 is a view illustrating one example of general configurations of the support member 260 and the container 110 according to a modification.

As illustrated in Fig. 5, the inner peripheral surface of the support member 260 and the outer peripheral surface of the cylindrical portion 111 of the container 110 may be inclined with respect to the centerline direction so that the detector 71 of the temperature sensor 70 easily comes into contact with the outer peripheral surface of the cylindrical portion 111 of the container 110. In other words, the diameter of the inner peripheral surface of the support member 260 is increased gradually from the first side toward the second side in the centerline direction, and the diameter of the outer peripheral surface of the cylindrical portion 111 of the container 110 is increased gradually from the first side toward the second side in the centerline direction. Consequently, when the container 110 is inserted to the inner side of the support member 260, the detector 71 of the temperature sensor 70 easily comes into contact with the outer peripheral surface of the cylindrical portion 111 of the container 110.

Note that the outer peripheral surface of the support member 260 may be also inclined with respect to the centerline direction so that the support member 260 has a uniform wall thickness. In addition, the inner peripheral surface of the cylindrical portion 111 of the container 110 may be also inclined with respect to the centerline direction so that the container 110 has a uniform wall thickness.

### <Third Embodiment>

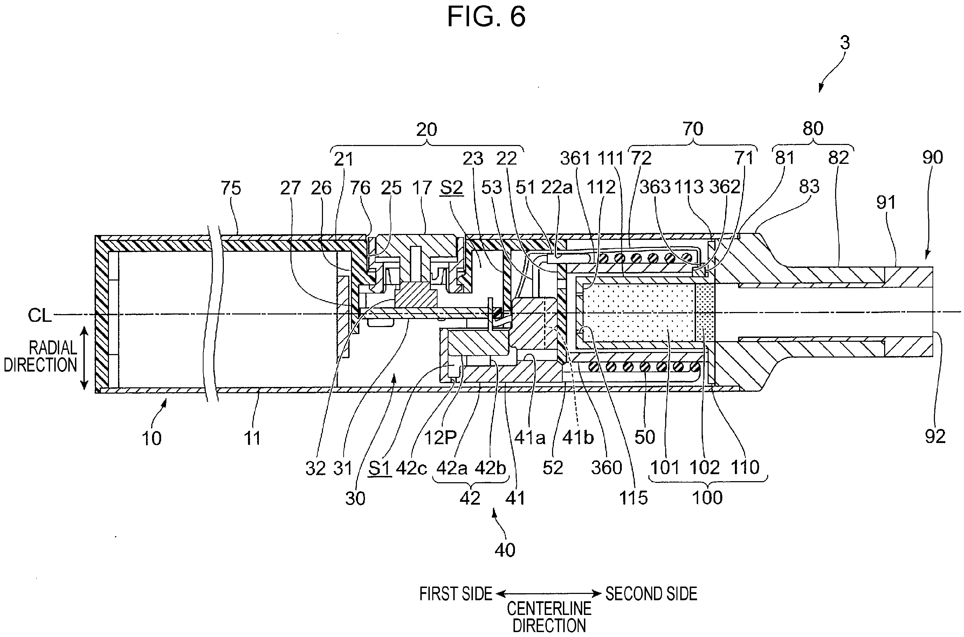

Fig. 6 is one example of a sectional view illustrating one example of a general configuration of an inhalation device 3 according to a third embodiment.

The inhalation device 3 according to the third embodiment differs from the inhalation device 2 according to the second embodiment in terms of the shape of a support member 360, which corresponds to the support member 260, and the position at which the temperature sensor 70 is provided. Hereinafter, differences from the second embodiment will be described. Identical signs are used for identical members in the second embodiment and the third embodiment, and detailed description thereof is omitted.

The support member 360 includes a cylindrical portion 361 having a cylindrical shape. An end portion of the cylindrical portion 361 on the second side in the centerline direction has a recess 362 that is recessed from the inner peripheral surface, and a through hole 363 extending in the radial direction and causing the recess 362 to be in communication with the outside of the cylindrical portion 361. Then, the detector 71 of the temperature sensor 70 is disposed in the recess 362. The lead wire 72 of the temperature sensor 70 passes through the through hole 363, passes on the second side in centerline direction with respect to the electromagnetic induction source 50 disposed on the outer side of the cylindrical portion 361, and passes through a gap between the outer side of the electromagnetic induction source 50 in the radial direction and the inner peripheral surface of the cover 75 and the through holes 22a of the disk-shaped portion 22. Two end portions provided at a tip of the lead wire 72 are each connected to the substrate 31.

As described above, the inhalation device 3 includes the support member 360 as one example of a non-magnetic member that has a tubular shape and that is disposed around the container 110, the container 110 having a tubular shape and the bottom 112 and being filled with the aerosol source, and the electromagnetic induction source 50 that is disposed around the support member 360 and that heats the container 110 by induction heating. In addition, the inhalation device 3 includes the temperature sensor 70 that includes the detector 71 that detects the temperature of the container 110, and the lead wire 72 that outputs a detection value, the detector 71 being disposed in the inside of the support member 360 to overlap a region in which the container 110 is provided in the centerline direction of the support member 360, the lead wire 72 passing on the outer side in the centerline direction with respect to the electromagnetic induction source 50 to extend from the detector 71 to the outer side of the electromagnetic induction source 50 in the radial direction.

In the inhalation device 3 that is configured as described, since the detector 71 is disposed in the centerline direction to overlap the region in which the container 110 is provided, the temperature sensor 70 detects the temperature of the cylindrical portion 111 of the container 110.

Therefore, according to the inhalation device 3, it is possible to suppress time and labor for maintenance of the inhalation device 3 and possible to highly accurately detect the temperature of the container 110 that heats the substrate 101 containing the aerosol source. In addition, since the temperature of the container 110 can be detected highly accurately, the controller 16 can suppress an excessive increase in the temperature difference between the temperature of the substrate 100 and a target temperature.

Further, since the temperature sensor 70 can detect the temperature of a portion close to a portion that tends to have a high temperature in the substrate 101 containing the aerosol source by detecting the temperature of the cylindrical portion 111 of the container 110 in which Joule heat is easily generated, the controller 16 can control the electric current that is to be supplied to the electromagnetic induction source 50 such that the temperature of the aerosol does not excessively increases.

Note that, to dispose the detector 71 such that the detector 71 of the temperature sensor 70 comes into contact with the outer peripheral surface of the cylindrical portion 111 of the container 110, it is desirable to provide, as described in the second embodiment, a projection protruding from the position of the detector 71 or from the inner peripheral surface of the cylindrical portion 361 of the support member 360. In addition, the inner peripheral surface of the support member 360 and the outer peripheral surface of the cylindrical portion 111 of the container 110 may be inclined with respect to the centerline direction.

### <Fourth Embodiment>

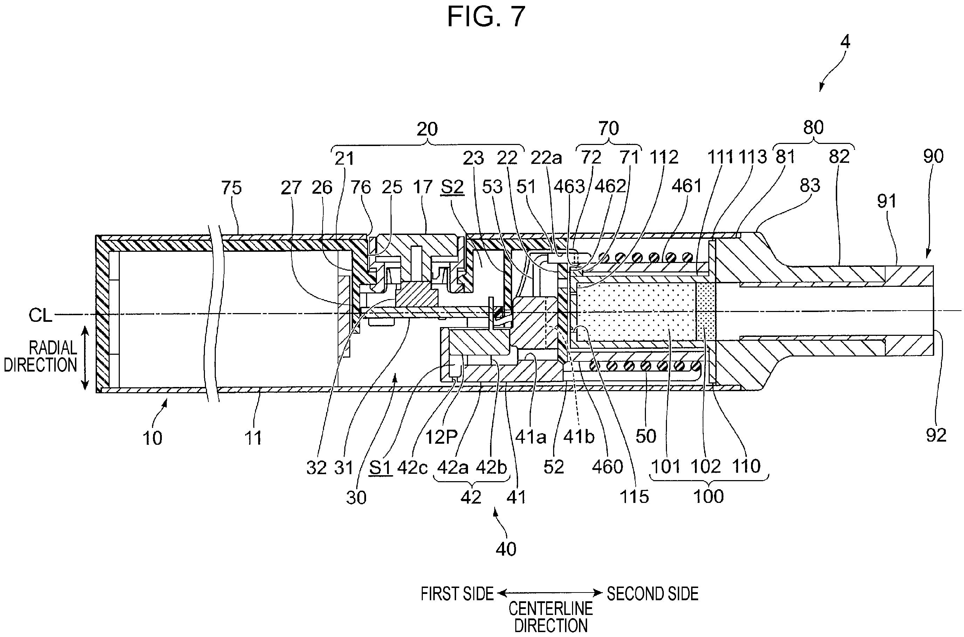

Fig. 7 is one example of a sectional view illustrating one example of a general configuration of an inhalation device 4 according to a fourth embodiment.

The inhalation device 4 according to the fourth embodiment differs from the inhalation device 2 according to the second embodiment in terms of the shape of a support member 460, which corresponds to the support member 260, and the position at which the temperature sensor 70 is provided. Hereinafter, differences from the second embodiment will be described. Identical signs are used for identical members in the second embodiment and the fourth embodiment, and detailed description thereof is omitted.

The support member 460 includes a cylindrical portion 461 having a cylindrical shape. An end portion of the cylindrical portion 461 on the first side in the centerline direction has a recess 462 that is recessed from the inner peripheral surface, and a through hole 463 extending in the radial direction and causing the recess 462 to be in communication with the outside of the cylindrical portion 461. Then, the detector 71 of the temperature sensor 70 is disposed in the recess 462. The lead wire 72 of the temperature sensor 70 passes through the through hole 463, passes on the first side in the centerline direction with respect to the electromagnetic induction source 50 disposed on the outer side of the cylindrical portion 461, and passes through the through holes 22a of the disk-shaped portion 22. Two end portions provided at a tip of the lead wire 72 are each connected to the substrate 31.

As described above, the inhalation device 4 includes the support member 460 as one example of a non-magnetic member that has a tubular shape and that is disposed around the container 110, the container 110 having a tubular shape and the bottom 112 and being filled with the aerosol source, and the electromagnetic induction source 50 that is disposed around the support member 460 and that heats the container 110 by induction heating. In addition, the inhalation device 4 includes the temperature sensor 70 that includes the detector 71 that detects the temperature of the container 110, and the lead wire 72 that outputs a detection value, the detector 71 being disposed in the inside of the support member 460 to overlap a region in which the container 110 is provided in the centerline direction of the support member 460, the lead wire 72 passing on the outer side in the centerline direction with respect to the electromagnetic induction source 50 to extend from the detector 71 to the outer side of the electromagnetic induction source 50 in the radial direction.

Similarly to the inhalation device 3, it is also possible in the inhalation device 4 that is configured as described above to suppress time and labor for maintenance of the inhalation device 4 and to highly accurately detect the temperature of the container 110 that heats the substrate 101 containing the aerosol source. In addition, since the temperature of the container 110 can be detected highly accurately, the controller 16 can suppress an excessive increase in the temperature difference between the temperature of the substrate 100 and a target temperature.

In addition, the inhalation device 4 further includes the substrate 31 to which an end portion of the lead wire 72 is connected, the detector 71 is disposed at an end portion of the support member 460 on the first side in the centerline direction, and the lead wire 72 extends at a position on the first side in the centerline direction, the position being a position closer than the electromagnetic induction source 50 to the substrate 31 in the centerline direction, to the outer side of electromagnetic induction source 50 in the radial direction. Therefore, the length of the lead wire 72 can be shortened.

Note that, to dispose the detector 71 such that the detector 71 of the temperature sensor 70 comes into contact with the outer peripheral surface of the cylindrical portion 111 of the container 110, it is desirable to provide, as described in the second embodiment, a projection protruding from the position of the detector 71 or from the inner peripheral surface of the cylindrical portion 461 of the support member 460. In addition, the inner peripheral surface of the support member 460 and the outer peripheral surface of the cylindrical portion 111 of the container 110 may be inclined with respect to the centerline direction.

### <Fifth Embodiment>

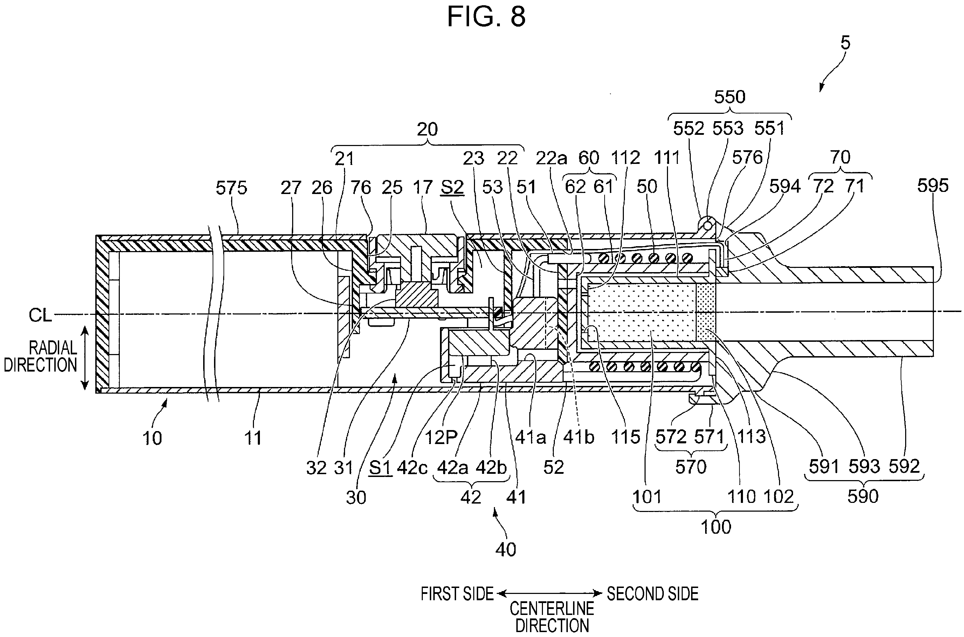

Fig. 8 is one example of a sectional view illustrating one example of a general configuration of an inhalation device 5 according to a fifth embodiment.

The inhalation device 5 according to the fifth embodiment differs from the inhalation device 1 according to the first embodiment in terms of the position at which the temperature sensor 70 is provided, the shape of a cover 575, which corresponds to the cover 75, and the shape of an opening-closing member 590, which corresponds to the end cap 80 and the mouthpiece 90. Hereinafter, differences from the first embodiment will be described. Identical signs are used for identical members in the first embodiment and the fifth embodiment, and detailed description thereof is omitted.

In the inhalation device 5, the cover 575 and the opening-closing member 590 are coupled to each other to constitute a hinge 550, and the opening-closing member 590 is configured to be openable and closable with respect to the cover 575. In addition, the cover 575 and the opening-closing member 590 constitute a suppression portion 570 that suppresses separation of the opening-closing member 590 from the cover 575 when the opening-closing member 590 closes an opening of the cover 575. Then, the detector 71 of the temperature sensor 70 is attached to the opening-closing member 590.

More specifically, the opening-closing member 590 includes a first cylindrical portion 591 that has a cylindrical shape and that is provided on the first side in the centerline direction, a second cylindrical portion 592 that has a cylindrical shape and that is provided on the second side in the centerline direction, and a connection portion 593 that connects the first cylindrical portion 591 and the second cylindrical portion 592 to each other.

The diameter of the outer peripheral surface of the second cylindrical portion 592 is smaller than the diameter of the outer peripheral surface of the first cylindrical portion 591, and the diameter of the inner peripheral surface of the second cylindrical portion 592 is identical to the diameter of the inner peripheral surface of the first cylindrical portion 591.

The diameter of the inner peripheral surface of the connection portion 593 is identical to the diameter of the inner peripheral surface of the first cylindrical portion 591, and the diameter of the outer peripheral surface of the connection portion 593 decreases gradually from the first cylindrical portion 591 toward the second cylindrical portion 592.

An end portion of the first cylindrical portion 591 on the first side in the centerline direction has a recess 594 that is recessed from an end surface and that extends in the radial direction. The detector 71 of the temperature sensor 70 is disposed at a portion of the recess 594 on the centerline CL side. The lead wire 72 of the temperature sensor 70 extends in the radial direction from the centerline CL side, passes on the outer side of the protrusion 113 of the container 110 and the electromagnetic induction source 50 in the radial direction, and passes through the through holes 22a of the disk-shaped portion 22. Two end portions provided at a tip of the lead wire 72 are each connected to the substrate 31.

An end portion of the first cylindrical portion 591 on the first side in the centerline direction is provided with a protrusion 551 that protrudes outward from the outer peripheral surface and that has a through hole through which a pin 553 passes. In addition, on the side opposite to the protrusion 551 with respect to the centerline CL, an end portion of the first cylindrical portion 591 on the first side in the centerline direction is provided with a hook 571 protruding outward from the outer peripheral surface and constituting the suppression portion 570.

The cover 575 has a cylindrical shape, and an end portion of the cover 575 on the second side in the centerline direction is provided with a protrusion 552 protruding outward from the outer peripheral surface and having a through hole through which the pin 553 passes. In addition, on the side opposite to the protrusion 552 with respect to the centerline CL, an end portion of the cover 575 on the second side in the centerline direction is provided with a projection 572 protruding outward from the outer peripheral surface and constituting the suppression portion 570.

The pin 553 passes through the through hole formed in the protrusion 551 of the opening-closing member 590 and the through hole formed in the protrusion 552 of the cover 575, thereby constituting the hinge 550. In addition, the hook 571 of the opening-closing member 590 is hooked on the projection 572 of the cover 575, thereby constituting the suppression portion 570.

As described above, the inhalation device 5 includes the electromagnetic induction source 50 that is disposed inside the cover 575, as one example of an insertion portion into which the container 110 having a tubular shape and the bottom 112 and filled with the aerosol source is inserted, and heats the container 110 by induction heating. In addition, the inhalation device 5 includes the opening-closing member 590 that changes between a state of covering an opening 576 of the cover 575 and a state of opening the opening 576 and has, in the state of covering, an air outlet hole 595 as one example of an outlet for aerosol generated as a result of heating of the aerosol source. In addition, the inhalation device 5 includes the temperature sensor 70 that is attached to the opening-closing member 590 and detects the temperature of the container 110.

Then, the container 110 includes the cylindrical portion 111 as one example of a tubular portion having a tubular shape, and the protrusion 113 disposed at a portion on the side opposite to the bottom 112 in the centerline direction to protrude outward from the cylindrical portion 111. The temperature sensor 70 includes the detector 71 that is disposed to face the protrusion 113 in the centerline direction and that detects the temperature of the container 110, and the lead wire 72 that passes the outer side of the electromagnetic induction source 50 in the radial direction to be connected to the substrate 31 and that outputs a detection value.

It is possible in the inhalation device 5 configured as described above to cause the detector 71 of the temperature sensor 70 to come into contact with the protrusion 113 of the container 110 and thus is possible to detect the temperature of the container 110 highly accurately.

Therefore, it is also possible in the inhalation device 5 to suppress time and labor for maintenance and to highly accurately detect the temperature of the container 110 that heats the substrate 101 containing the aerosol source. In addition, since the temperature of the container 110 can be detected highly accurately, the controller 16 can suppress an excessive increase in the temperature difference between the temperature of the substrate 100 and a target temperature. As a result, according to the inhalation device 5, it is possible to cause the temperature of the substrate 101 packed in the container 110 to be an appropriate temperature.

Note that, although the opening-closing member 590 corresponds to the end cap 80 and the mouthpiece 90, the opening-closing member 590 is not limited in particular to such a form. For example, the opening-closing member 590 may correspond to the end cap 80 and may be configured such that a member (for example, the mouthpiece 90) to be held in the mouth of a user during inhalation is attachable to the opening-closing member 590.

### <Sixth Embodiment>

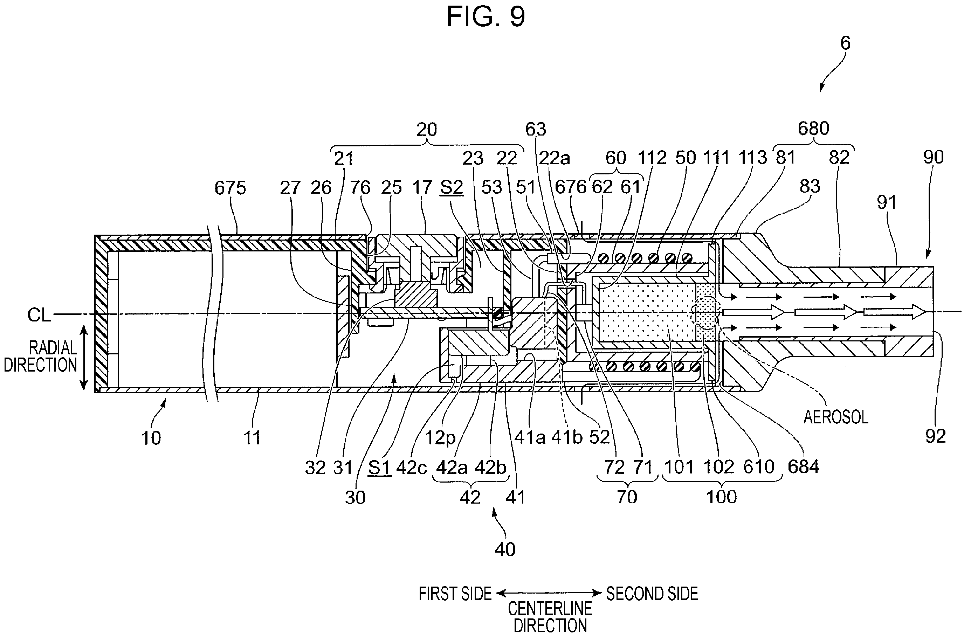

Fig. 9 is one example of a sectional view illustrating one example of a general configuration of an inhalation device 6 according to a sixth embodiment.

The inhalation device 6 according to the sixth embodiment differs from the inhalation device 1 according to the first embodiment in terms of a flow path for air to be inhaled by a user Hereinafter, differences from the first embodiment will be described. Identical signs are used for identical members in the first embodiment and the sixth embodiment, and detailed description thereof is omitted.

Differently from the container 110 according to the first embodiment, the through holes 115 are not formed in a container 610, which corresponds to the container 110 according to the first embodiment, according to the sixth embodiment.

A cover 675, which corresponds to the cover 75 according to the first embodiment, according to the sixth embodiment has a communication hole 676 at a portion that is on the second side in the centerline direction with respect to the holder 20 and that is also on the first side in the centerline direction with respect to the protrusion 113 of the container 110. The communication hole 676 extends through the cover 675 in the radial direction and causes the outside and the inside of the cover 675 to be in communication with each other. As an example, a plurality of the communication holes 676 are formed in the circumferential direction.

In addition, an end cap 680, which corresponds to the end cap 80 according to the first embodiment, according to the sixth embodiment has, on an end portion of the flange 83 on the first side in the centerline direction, a groove 684 that is recessed from an end surface toward the second side. The groove 684 is formed over the entire region of the flange 83 from the outer peripheral portion to the inner peripheral portion in the radial direction. As an example, a plurality of the grooves 684 are formed in the circumferential direction.

### (Operation of Inhalation Device 6)

By inserting the container 610 filled with the aerosol source into the support member 60 and induction heating the container 610 by using the electromagnetic induction source 50, a user can cause the inhalation device 6 configured as described above to be in a state in which aerosol can be inhaled. Then, when inhalation is performed by a user, as illustrated in Fig. 9, the aerosol generated as a result of heating of the aerosol source accommodated in the container 610 flows in through the communication hole 676 formed in the cover 675 and mixes with air that has passed through a gap between the protrusion 113 of the container 610 and the cover 675 and through the groove 684 of the end cap 680. Then, a mixture fluid of the aerosol and the air passes through the inside of the mouthpiece 90 and is conveyed to the air outlet hole 92 formed in the mouthpiece 90.

Then, due to having no through holes 115 formed in the bottom 112 of the container 610, the inhalation device 6 configured as described above can exert, in addition to the effect exerted by the inhalation device 1 according to the above-described first embodiment, an additional effect of causing the heated aerosol source not to partially remain on the inner side of the electromagnetic induction source 50 after the container 610 is taken out.

Note that, similarly to the inhalation device 6 according to the sixth embodiment, each of the inhalation devices 2 to 5 according to the second to fifth embodiments may be configured such that the through holes 115 are not formed in the bottom 112 of the container 110 and that the aerosol generated as a result of heating of the aerosol source accommodated in the container 110 is caused to flow out after mixing with air that has flowed in through a communication hole, which is formed at a portion of the cover 75 on the second side in the centerline direction with respect to the holder 20 and also on the first side in the centerline direction with respect to the protrusion 113 of the container 110.

In addition, while having a configuration including the single temperature sensor 70, each of the inhalation devices 1 to 5 according to the above-described first to fifth embodiments is not limited in particular to such a form and may include a plurality of the temperature sensors 70. In addition, when a plurality of the temperature sensors 70 are included, the temperature sensors 70 may be disposed at a plurality of positions in each of the inhalation devices 1 to 5. For example, the inhalation device 2 according to the second embodiment may include a plurality of the temperature sensors 70 such that the detector 71 of each of the temperature sensors 70 is disposed at, in addition to a central portion of the cylindrical portion 261 of the support member 260 in the centerline direction, a position of at least either one of an end portion (a position similar to that in the inhalation device 4) on the first side in the centerline direction and an end portion (a position similar to that in the inhalation device 3) on the second side in the centerline direction. By including a plurality of the temperature sensors 70, it is possible to further highly accurately detect the temperature of the container 110.

### Reference Signs List

1, 2, 3, 4, 5, 6 inhalation device
10 power supply unit
16 controller
20 holder
21 base portion
31 substrate
50 electromagnetic induction source
60, 260, 360, 460 support member
70 temperature sensor
71 detector
72 lead wire
75, 575, 675 cover
80 end cap
90 mouthpiece
92, 595 air outlet hole
110, 610 container
111 cylindrical portion
112 bottom
113 protrusion
262, 362, 462, 594 recess
263, 363, 463 through hole
576 opening
590 opening-closing member

## Claims

1. An inhalation device comprising:
an electromagnetic induction source that is disposed at a portion into which a container is to be inserted, and that heats a side surface of the container by induction heating, the container having a tubular shape and a bottom and being filled with an aerosol source;
a temperature sensor that is disposed at a position facing the bottom of the container and that detects a temperature of the container; and
an abutting member that causes the bottom of the container to abut onto the temperature sensor.

2. An inhalation device comprising:
a non-magnetic member that has a tubular shape and that is disposed around a container, the container having a tubular shape and a bottom and being filled with an aerosol source;
an electromagnetic induction source that heats the container by induction heating, the electromagnetic induction source being constituted by a conductive wire that is wound into a helical shape around an outer peripheral surface of the non-magnetic member; and
a temperature sensor that includes a detector that detects a temperature of the container, and a lead wire that outputs a detection value, the detector being disposed in an inside of the non-magnetic member to overlap a region in which the container is provided in a centerline direction of the non-magnetic member, the lead wire passing between portions of the conductive wire of the electromagnetic induction source to extend from the detector to an outer side of the electromagnetic induction source.

3. An inhalation device comprising:
a non-magnetic member that has a tubular shape and that is disposed around a container, the container having a tubular shape and a bottom and being filled with an aerosol source;
an electromagnetic induction source that is disposed around the non-magnetic member and that heats the container by induction heating; and
a temperature sensor that includes a detector that detects a temperature of the container, and a lead wire that outputs a detection value, the detector being disposed in an inside of the non-magnetic member to overlap a region in which the container is provided in a centerline direction of the non-magnetic member, the lead wire passing an outer side in the centerline direction with respect to the electromagnetic induction source to extend from the detector to the outer side of the electromagnetic induction source.

4. The inhalation device according to claim 3, further comprising:
a substrate to which an end portion of the lead wire is connected,
wherein, at a position closer than the electromagnetic induction source to the substrate in the centerline direction, the lead wire extends to the outer side of the electromagnetic induction source.

5. An inhalation device comprising:
an electromagnetic induction source that heats a container by induction heating, the container having a tubular shape and a bottom and being filled with an aerosol source, the electromagnetic induction source being disposed at an insertion portion into which the container is to be inserted;
an opening-closing member that changes between a state of covering an opening of the insertion portion and a state of opening the opening, the opening-closing member having, in the state of covering, an outlet for aerosol that is generated as a result of heating of the aerosol source; and
a temperature sensor that is attached to the opening-closing member and that detects a temperature of the container.

6. The inhalation device according to claim 5,
wherein the container includes a tubular portion that has a tubular shape, and a protrusion at a portion on a side opposite to the bottom in a centerline direction, the protrusion protruding outward from the tubular portion, and
wherein the temperature sensor includes a detector that detects a temperature of the container, and a lead wire that outputs a detection value, the detector being disposed to face and to come into contact with the protrusion in the centerline direction, the lead wire passing an outer side of the electromagnetic induction source and being connected to a substrate.
